# EUROPEAN PATENT APPLICATION

(11) **EP 1 741 786 A1**
(43) Date of publication of application: **10.01.2007**
(21) Application number: 05727402.9
(22) Date of filing: 28.03.2005
(51) Int. Cl.: C12N 15/63, C12N 5/10, C12Q 1/02

(54) **METHOD OF SEPARATING PRECUSROR CELLS OF LIVER CELLS, ENDOTHELIAL CELLS OR HEMATOPOIETIC CELLS FROM CELL MASS**

(30) Priority: 29.03.2004 JP 2004096744
(71) Applicant: Sugiyama, Haruo, Minoo-shi, Osaka 562-0036 (JP)
(72) Inventor: SUGIYAMA, Haruo, Minoo-shi, Osaka 5620036 (JP); HOSEN, Naoki, Minoo-shi, Osaka 5620031 (JP); HASTIE, N., D., Edinburgh (GB)
(74) Representative: Vossius & Partner
(86) International application number: PCT/JP2005/005787
(87) International publication number: WO 2005/093076

(57) **Abstract**

The present inventors succeeded in FACS-sorting viable WT1-expressing cells, and also discovered that WT1 expression in mouse fetal liver cells serves as a common molecular marker of hepatic, endothelial, and hematopoietic progenitor cells. Based on the present invention, hepatic, endothelial, and hematopoietic progenitor cells can be separated or detected using the WT1 gene expression level as an indicator.

## Description

### Technical Field

The present invention relates to methods for separating progenitor cells from cell populations.

### Background Art

Various types of stem cells and progenitor cells exist in the bodies of animals. Tissue-specific stem cells and progenitor cells are present in each type of tissue, and many daughter cells are produced to maintain the tissues.

So far, many researchers have committed much effort to isolating many types of tissue-specific stem cells and progenitor cells, and two different types of strategies have been used to this end: One strategy is cell separation based on the use of a combination of surface antigens (for example c-kit⁺, Thy. 1^{lo}, Sca-1⁺, and 1in⁻), which is aimed at isolating hematopoietic stem cells or progenitor cells (Non-Patent Document 1). The other strategy is to label cells by expressing a particular gene using a reporter gene (for example, a gene produced by linking green fluorescent protein (GFP) to a promoter or an enhancer of a gene specific to stem cells or progenitor cells, such as nestin enhancer-GFP reporter which is used to separate neural stem cells (Non-Patent Document 2)).

In both strategies, many marker molecules are specific to a single lineage of stem cells or progenitor cells. Molecular markers common to such cells regardless of lineage have not been reported.
[Non-Patent Document 1] Morrison S. J., et al. The biology of hematopoietic stem cells. Annu. Rev. Cell Dev. Biol. 1995; 11: 35-71
[Non-Patent Document 2] Roy NS, et al. In vitro neurogenesis by progenitor cells isolated from the adult human hippocampus. Nat. Med. 2000; 6: 271-7.
[Non-Patent Document 3] Call KM, et al. Isolation and characterization of a zinc finger polypeptide gene at the human chromosome 11 Wilms' tumor locus. Cell. 1990; 60: 509-520
[Non-Patent Document 4] Gessler M, et al. Homozygous deletion in Wilms tumors of a zinc-finger gene identified by chromosome jumping. Nature. 1990; 343: 774-778
[Non-Patent Document 5] Menke, A.L., et al. The Wilms' tumor 1 gene: Oncogene or tumor suppressor gene? Int. Rev. Cytol. 1998; 181: 51-212.
[Non-Patent Document 6] Larsson SH, et al. Subnuclear localization of WT1 in splicing or transcription factor domains is regulated by alternative splicing. Cell. 1995; 81: 391-401.
[Non-Patent Document 7] Kreidberg, J.A., et al. WT-1 is required for early kidney development. Cell. 1993; 74, 679-691.
[Non-Patent Document 8] Inoue, K., et al. WT1 as a new prognostic factor and a new marker for the detection of minimal residual disease in acute leukemia. Blood. 1994; 84: 3071-3079.
[Non-Patent Document 9] Sugiyama, H. Wilms' tumor gene WT1: its oncogenic function and clinical application. Int. J. Hematol. 2002, 73: 177-87.
[Non-Patent Document 10] Menssen HD, et al. Wilms tumor gene (WT1) expression as a panleukemic marker. Int J Hematol. 2002; 76: 103-9.
[Non-Patent Document 11] Loeb DM, et al. The role of WT1 in oncogenesis: tumor suppressor or oncogene? Int. J. Hematol. 2002; 76: 117-26.
[Non-Patent Document 12] Oji Y, et al. Overexpression of the Wilms' tumor gene WT1 in primary thyroid cancer. 2003; 94: 606-611.
[Non-Patent Document 13] Oji Y, et al. Overexpression of the Wilms' tumor gene WT1 in colorectal adenocarcinoma. Cancer Sci. 2003; 94: 712-7.
[Non-Patent Document 14] Oji Y, et al. Overexpression of the Wilms' tumor gene WT1 in head and neck squamous cell carcinoma. Cancer Sci. 2003; 94: 523-9.
[Non-Patent Document 15] Inoue K, et al. Aberrant overexpression of the Wilms tumor gene (WT1) in human leukemia. Blood. 1997; 89: 1405-1412
[Non-Patent Document 16] Hosen N, et al. Very low frequencies of human normal CD34+ haematopoietic progenitor cells express the Wilms' tumor gene WT1 at levels similar to those in leukaemia cells. Br. J. Haematol. 2002; 116: 409-20.
[Non-Patent Document 17] Moore AW, et al. YAC transgenic analysis reveals Wilms' tumour 1 gene activity in the proliferating coelomic epithelium, developing diaphragm and limb. Mech. Dev. 1998; 79: 169-84
[Non-Patent Document 18] Fiering SN et al. Improved FACS-Gal: flow cytometric analysis and sorting of viable eukaryotic cells expressing reporter gene constructs. Cytometry. 1991; 12: 291-301.
[Non-Patent Document 19] Li H, et al. The lck promoter-driven expression of the Wilms tumor gene WT1 blocks intrathymic differentiation of T-lineage cells. Int. J. Hematol. 2003; 77: 463-70.
[Non-Patent Document 20] Suzuki A, et al. Flow-cytometric separation and enrichment of hepatic progenitor cells in the developing mouse liver. Hepatology. 2000; 32: 230-9.
[Non-Patent Document 21] Asahara T, et al. Isolation of putative progenitor endothelial cells for angiogenesis. Science. 1997; 275: 964-7.
[Non-Patent Document 22] Murayama A, et al. Flow cytometric analysis of neural stem cells in the developing and adult mouse brain. J. Neurosci. Res. 2002; 69: 837-47.
[Non-Patent Document 23] Arai F, et al. Mesenchymal stem cells in perichondrium express activated leukocyte cell adhesion molecule and participate in bone marrow formation. J. Exp. Med. 2002; 195: 1549-63.

### Disclosure of the Invention

The present invention was achieved in view of the above circumstances. An objective of the present invention is to provide molecular markers that are common to various progenitor cells, regardless of cell lineage. A further objective of the present invention is to provide methods for separating progenitor cells from cell populations by using such molecular markers that are common to various progenitor cells.

Wilms' tumor gene WT1 has been identified as a gene responsible for Wilms' tumor, a type of childhood liver cancer (Non-Patent Documents 3 and 4). The WT1 gene encodes a transcription factor comprising a zinc finger motif, and regulates the expression of many genes relating to growth, differentiation, and apoptosis (Non-Patent Document 5). The WT1 gene also plays an important role in mRNA splicing (Non-Patent Document 6). Analysis of WT1 gene-disrupted mice has shown that the WT1 gene plays a definitive role in early urogenital development (Non-Patent Document 7). The present inventors (Non-Patent Documents 8 and 9) and other researchers (Non-Patent Document 10) have previously reported that expression of the wild-type WT1 gene is strong in almost all leukemia samples, regardless of disease subtype.

WT1 is also overexpressed in many types of solid tumors such as lung cancer (Non-Patent Document 11), breast cancer (Non-Patent Document 11), thyroid cancer (Non-Patent Document 12), colonic cancer (Non-Patent Document 13), head and neck squamous cell cancer (Non-Patent Document 14), renal cell carcinoma (Non-Patent Document 11), and malignant mesothelioma (Non-Patent Document 11). In contrast, WT1 genes are expressed at very low levels in many types of normal tissue (Non-Patent Documents 12-16). WT1 is expressed at low levels in normal human hematopoietic cells, and that expression is limited to immature CD34+ progenitor cells (Non-Patent Document 15).

Recently, the present inventors elucidated that the frequency of "WT1-expressing progenitor cells" in normal hematopoietic cells is very low (not more than 1.2% of CD34+ cells) (Non-Patent Document 16). Based on these results, the present inventors hypothesized that "WT1-expressing progenitor cells" may also be present at very low frequencies in many other types of tissues.

Fetal liver comprises progenitor cells of various cell lineages, such as hepatocytes, endothelial cells, and hematopoietic cells. Therefore, fetal liver is an ideal organ for verifying the above-mentioned hypothesis. The present inventors identified and collected WT1-expressing fetal liver cells, and verified whether there were many WT1-expressing cells in hepatic, endothelial, and hematopoietic progenitor cells.

The results showed that mouse fetal liver cells can be clearly separated into three different subpopulations according to their WT1 gene expression level; that many endothelial progenitor cells are comprised among WT1++ fetal liver cells; and that many hematopoietic progenitor cells are present among cells with an intermediate level of WT1 expression. Further, the present inventors examined whether or not WT1-expressing cells are present in other fetal tissues such as fetal brain and fetal limb. This revealed that cells expressing high levels of WT1 exist at a low frequency among fetal brain cells and mesenchymal cells.

Specifically, the present inventors successfully accomplished FACS sorting of viable WT1-expressing cells, and also showed for the first time that WT1 expression in mouse fetal liver cells can serve as a common molecular marker of hepatic, endothelial, and hematopoietic progenitor cells, thus completing the present invention.

More specifically, the present invention provides:
[1] a method for separating a hepatic, endothelial, or hematopoietic progenitor cell from a cell population, wherein the method comprises the steps of:
   a) detecting the expression of a WT1 gene in a cell in a cell population, and
   b) separating the cell in which expression of the WT1 gene was detected;
[2] a method for simultaneously separating at least two progenitor cells from a cell population, wherein the progenitor cells are selected from hepatic, endothelial, and hematopoietic progenitor cells, and wherein the method comprises the steps of:
   a) detecting the expression of a WT1 gene in a cell in a cell population comprising at least two progenitor cells, selected from hepatic, endothelial, and hematopoietic progenitor cells, and
   b) separating the cells in which expression of the WT1 gene was detected;
[3] the method of [1] or [2], wherein expression of the WT1 gene is detected by using expression of a WT1 gene or of a reporter gene linked to a WT1 promoter as an indicator;
[4] the method of [3], wherein the reporter gene is a lacZ gene or GFP gene, and expression of the reporter gene is detected by a FACS assay; and
[5] the method of any one of [1] to [4], wherein a hepatic progenitor cell or an endothelial progenitor cell is separated when the expression level of the WT1 gene is in the range of 2.21 (±1.62) x 10⁻² (when expression of the WT1 gene in a K562 leukemia cell line is defined as 1), and a hematopoietic progenitor cell is separated when the expression level of the WT1 gene is in the range of 3.54 (±3.39) x 10⁻⁴ (when expression of the WT1 gene in a K562 leukemia cell line is defined as 1).

### Brief Description of the Drawings

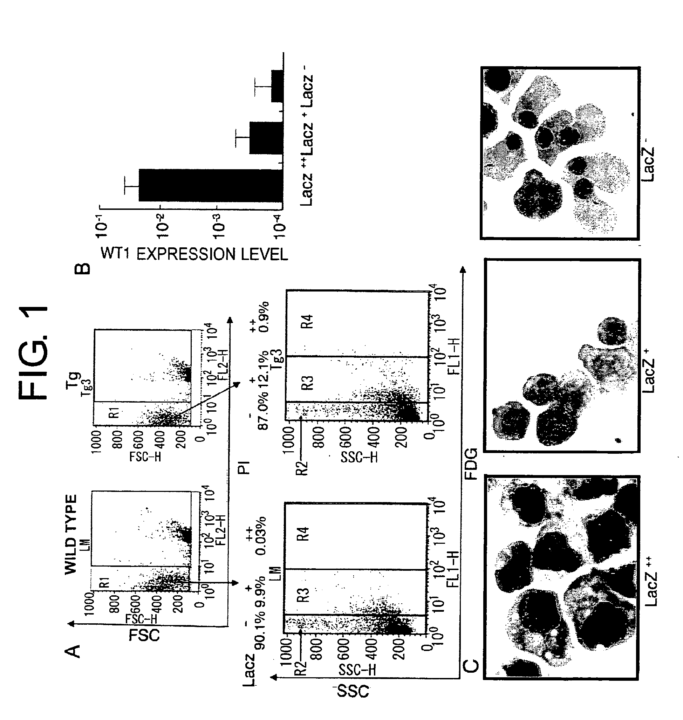

Fig. 1 consists of diagrams (A, B) and photographs (C), which show that mouse fetal liver cells can be separated into three different subpopulations according to the level of WT1 gene expression. A. Representative FACS profiles of the fetal liver cells of E12.5 embryos of WT470LZ Tg and wild-type mice when the cells were incubated with FDG The upper diagrams depict the FACS profiles of propidium iodide (PI) staining for forward scatter. Dead cells were excluded from analyses based on PI uptake. In the lower diagrams, the X-axis indicates the β-galactosidase activity (measured as fluorescence) of FACS-Gal-labeled fetal liver cells. The Y-axis indicates side scatter. The gating windows and percentages of cells in each window are shown for the lacZ++, lacZ+, and lacZ-fractions. The percentages indicate the proportion of each cell population in terms of the total number of viable cells (PI-). B. As indicated elsewhere (Non-Patent Document 19), 3000 lacZ++ cells, lacZ+ cells, and lacZ- cells were FACS-sorted, and their WT1 expression levels were analyzed by quantitative real time RT-PCR. Each value represents the mean ± standard error (SE) obtained from three independent experiments. C. Morphological characteristics of lacZ++ cells, lacZ+ cells, and lacZ- cells. Fetal liver cells of WT470LZ Tg mice were FACS-sorted to obtain lacZ++ cells, lacZ+ cells, and lacZ- cells. May-Grunwald-Giemsa-stained cytocentrifugal preparations of sorted cells are shown.

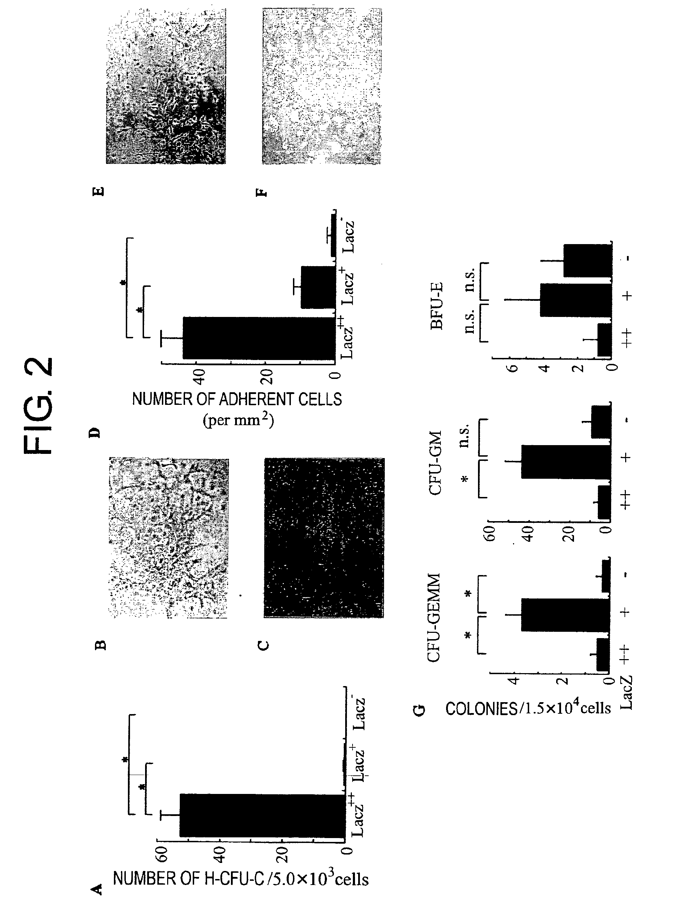

Fig. 2 consists of diagrams (A, D, G) and photographs (B, C, E, F) that show that WT1-expressing fetal liver cells comprise a large number of hepatic (A, B, C), endothelial (D, E, F), and hematopoietic (G) progenitor cells. A. The number of H-CFU-C colonies produced from lacZ++ cells, lacZ+ cells, and lacZ- cells per 5000 cells. Each value represents the mean ± standard error (SE) obtained from three independent experiments. B. Representative H-CFU-C colonies derived from lacZ++ cells are shown. C. These colonies were positively stained with anti-albumin antibody. D. FACS-sorted fetal liver cells were plated onto fibronectin-coated 35-mm culture plates at a density of 1.5 x 10⁴ cells per plate. The diagram shows the number of adherent cells per mm² produced from lacZ++, lacZ +, and lacZ- fetal liver cells after four days of EPC culturing. Each value represents the mean ± SE obtained from three independent experiments. E. Adherent cells four days after plating lacZ+ fetal liver cells onto fibronectin in EPC medium are shown. F. After co-culturing the adherent cells with acLDL-Dil for four hours, uptake of acLDL-Dil by the cells was observed. G. The frequency of CFU-GEMM on Day 12, and the frequency of CFU-GM and of BFU-E on Day 9 in methylcellulose agar cultures of sorted lacZ++, lacZ+, and lacZ- fetal liver cells derived from WT470LZ Tg and wild-type mice are shown. Each value represents the mean ± SE obtained from three independent experiments.

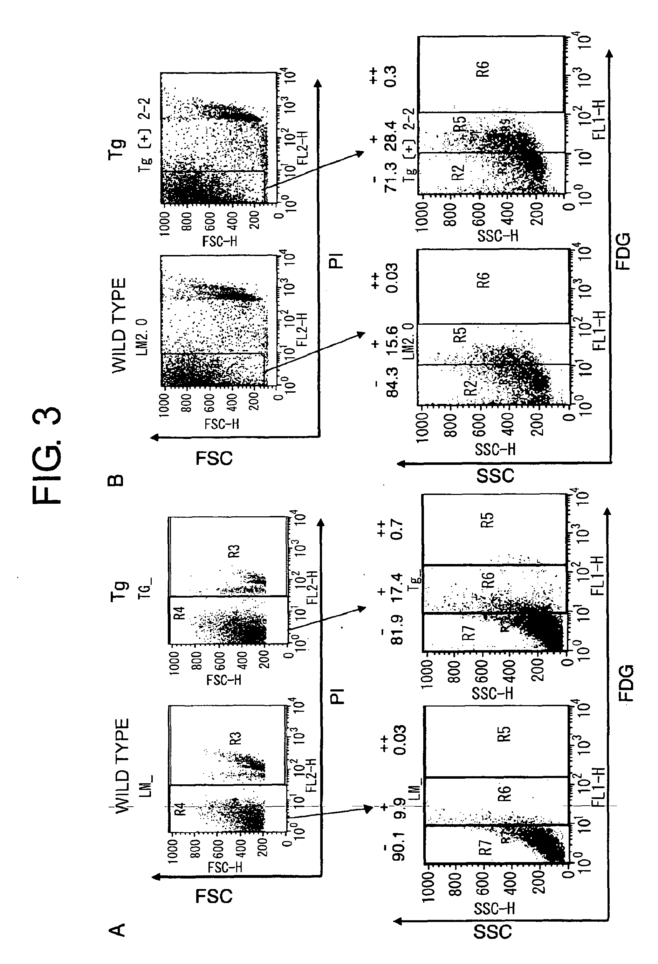

Fig. 3 shows that "WT1-expressing cells" are present at a very low frequency in (A) fetal brain and (B) fetal limb. Cell suspensions were prepared as described previously (Non-Patent Documents 22 and 23). The upper diagrams show the FACS profile as a plot of PI staining for forward scatter. Dead cells were excluded from the analysis based on the results of PI uptake. In the lower diagrams, the X-axis indicates the β-galactosidase activity of FACS-Gal-labeled fetal liver cells (measured as fluorescence). The Y-axis indicates side scatter.
The cells were separated into three subpopulations according to β-galactosidase activity, as with the fetal liver cells. The gating windows and percentages of cells in each window are shown for the lacZ++, lacZ+, and lacZ-fractions. The percentages indicate the proportion of each population in terms of the total number of viable cells (PI-). A. Representative FACS profiles of fetal brain cells of E14.5 embryos of WT470LZ Tg and wild-type mice when the cells were incubated with FDG. B. Representative FACS profiles of fetal limb cells of E16.5 embryos of WT470LZ Tg and wild-type mice when the cells were incubated with FDG.

### Best Mode for Carrying Out the Invention

The present invention provides methods for separating progenitor cells from cell populations using WT1 gene expression as an indicator.

In the present invention, the term "WT1 gene" refers to genes that encode a transcription factor comprising a zinc finger motif, which have been identified as genes that cause Wilms' tumor. The origin of the "WT1 gene" is not limited, and the "WT1 gene" includes WT1 genes derived from humans, mice, and other vertebrates. Nucleotide sequences of the WT1 genes have been elucidated in the animal species below. Currently unidentified homologs in other animal species can also be isolated based on such known nucleotide sequence information. For example, methods for isolating homologs using hybridization and PCR are well known.
Human WT1: NM_024425
Mouse WT1: NM_144783

Progenitor cells separated by the methods of the present invention are not limited so long as the cells can express a WT1 gene, but for example, hepatic, endothelial, or hematopoietic progenitor cells are preferred as target cells. Separation of progenitor cells includes the steps of: (a) detecting WT1 gene expression in a cell belonging to a cell population from which progenitor cells are to be separated; and (b) separating the cell in which WT1 gene expression was detected.

Any cell population that may comprise desired progenitor cells can be used as a cell population comprising such progenitor cells. More specifically, desired progenitor cells can be separated by targeting cell populations such as the following:
Hepatic progenitor cells: fetal liver, excised liver, and human ES cells
Endothelial progenitor cells: bone marrow, umbilical, and human ES cells
Hematopoietic progenitor cells: bone marrow, umbilical, and human ES cells

In particular, fetal liver cells can be used as cell populations comprising all of these progenitor cells. These cell populations may be fractioned in advance, depending on their purpose. For example, cell populations from which undesired cell fractions have been removed in advance can be used as cell populations in the present invention.

The present inventors discovered that WT1 genes can be used as common markers for hepatic, endothelial, and hematopoietic progenitor cells. Therefore, by using WT1 gene expression as an indicator, at least two types of progenitor cells, selected from among hepatic, endothelial, and hematopoietic progenitor cells, can be simultaneously separated from a cell population comprising at least two types of progenitor cells, selected from among hepatic, endothelial, and hematopoietic progenitor cells. More specifically, the present invention relates to the use of WT1 genes as markers for any one progenitor cell selected from the group consisting of hepatic progenitor cells, endothelial progenitor cells, and hematopoietic progenitor cells.

The methods for detecting WT1 gene expression in cells are not particularly limited, so long as they are methods that can detect viable cells. Preferably, they are detection methods in which expression of a WT1 gene or reporter gene linked to a promoter of a WT1 gene are used as an indicator. Therefore, in the present invention, the expression level of a reporter gene that is expressed under the control of a WT1 gene promoter is included in the expression level of the WT1 genes. In fact, as described below, the expression levels of reporter genes expressed under the control of a WT1 gene promoter have been confirmed to match the expression patterns of WT1 genes. The reporter genes are not particularly limited, so long as their expression can be detected. For example, by using a lacZ gene or GFP gene as the reporter gene, reporter gene expression can be easily detected and separated by FACS assay.

For example, when using GFP gene as a reporter gene, transgenic animals carrying a vector in which the GFP gene is operably linked downstream of a WT1 gene promoter are produced (so that the GFP gene is expressed in host cells), and then the desired cells can be identified from among cells collected from such animals by using GFP gene expression as an indicator. Furthermore, animals in which the GFP gene is knocked-in downstream of a WT1 gene promoter region are produced, and then the desired cells can be identified from among cells collected from such animals by using GFP gene expression as an indicator.

Alternatively, WT1-expressing cells can be collected as viable cells by introducing a WT1 reporter into cells using lentiviruses. WT1 reporters are constructs that comprise a WT1 promoter and enhancer and express reporter genes. WT1 reporters that use green fluorescent gene (GFP) as a reporter gene are known (Hosen N. et al. Leukemia 2004 18(3) 415-9). Furthermore, antibodies that bind specifically to WT1-positive cells can be produced using WT1-positive cells separated according to the present invention. Antibodies specific to WT1-positive cells obtained in this manner can be used to separate, detect, and identify WT1-positive cells. For example, WT1-positive cells can be separated by cell sorting using anti-WT1-positive cell antibodies.

When separating hepatic progenitor cells or endothelial progenitor cells according to the present invention, the WT1 gene expression levels can be compared by using indicators such as the following: That is, hepatic progenitor cells or endothelial progenitor cells can be separated by selecting cells in which the expression level of a WT1 gene is in the range of 2.21 (±1.62) x 10⁻² when the expression level of the WT1 gene in leukemia cell line K562 is defined as 1. Alternatively, hematopoietic progenitor cells can be separated by selecting cells in which the expression level of a WT1 gene is in the range of 3.54 (±3.39) x 10⁻⁴ when the expression level of the WT1 gene in leukemia cell line K562 is defined as 1. More specifically, the present invention relates to methods for separating hepatic progenitor cells or endothelial progenitor cells, wherein the methods comprise the following steps:
i. measuring a WT1 gene expression level in a cell population comprising hepatic progenitor cells and/or endothelial progenitor cells;
ii. comparing the WT1 gene expression level measured in (i) with the WT1 gene expression level in leukemia cell line K562; and
iii. separating cells as hepatic progenitor cells and endothelial progenitor cells, in which the WT1 gene expression level is in the range of 2.21 (±1.62) x 10⁻², when the WT1 gene expression level in leukemia cell line K562 is defined as 1.

The present invention also relates to methods for separating hematopoietic cells, wherein the methods comprise the following steps:
i. measuring a WT1 gene expression level in a cell population comprising hematopoietic progenitor cells;
ii. comparing the WT1 gene expression level measured in (i) with the WT1 gene expression level in leukemia cell line K562; and
iii. separating cells as hematopoietic progenitor cells, in which the WT1 gene expression level is in the range of 3.54 (±3.39) x 10⁻⁴, when the WT1 gene expression level in leukemia cell line K562 is defined as 1.

In the present invention, gene expression levels can be compared by any method. For example, gene expression levels can be quantitatively compared based on the expression intensity of a reporter gene.

In the present invention, the expression level of leukemia cell line K562 was presented as a control against which the WT1 gene expression level was compared. Leukemia cell line K562 can be obtained as ATCC Accession No. CCL-243. Alternatively, the WT1 gene expression level in cells other than leukemia cell strain K562 can be used to compare WT1 gene expression levels in the present invention. For example, even cells other than K562 can be used as comparative controls of the present invention if WT1 gene expression levels can be corrected by pre-determining the ratio of WT1 gene expression levels in these cells against the expression level in leukemia cell line K562. Such embodiments, in which WT1 expression levels in alternative cells are compared, are included in the present invention so long as the expression levels can be represented in terms of the expression level in leukemia cell line K562.

Progenitor cells separated according to the present invention can be widely used, especially in the field of regenerative medicine. The hepatic progenitor cells, endothelial progenitor cells, and hematopoietic progenitor cells that can be separated by the present invention are all important cells in the field of regenerative medicine. Therefore, discovering cell populations that may comprise such cells is an important task in regenerative medicine that uses such progenitor cells. The present invention showed that WT1 genes can serve as markers for hepatic progenitor cells, endothelial progenitor cells, and hematopoietic progenitor cells. Therefore, these progenitor cells can be detected using WT1 gene expression as an indicator.

More specifically, the present invention relates to methods for identifying any cells selected from the group consisting of hepatic progenitor cells, endothelial progenitor cells, and hematopoietic progenitor cells, wherein the methods comprise the following steps:
i. measuring the expression level of a WT1 gene in cells; and
ii. identifying those cells in which WT1 gene expression was detected as cells selected from the group consisting of hepatic progenitor cells, endothelial progenitor cells, and hematopoietic progenitor cells.

In the present invention, each of the progenitor cells can be specifically detected by quantitatively comparing their WT1 gene expression levels. More specifically, if the WT1 gene expression level is in the range of 2.21 (±1.62) x 10⁻² when the WT1 gene expression level in leukemia cell line K562 is defined as 1, the cells can be detected as hepatic progenitor cells and endothelial progenitor cells. If the WT1 gene expression level is in the range of 3.54 (±3.39) x 10⁻⁴ when the WT1 gene expression level in leukemia cell line K562 is defined as 1, the cells can be detected as hematopoietic progenitor cells. Cell populations comprising cells confirmed to express a WT1 gene may be used as cell populations for obtaining hepatic progenitor cells, endothelial progenitor cells, and hematopoietic progenitor cells. Cell populations in which these progenitor cells have been detected are preferable as materials for obtaining each of these progenitor cells.

In the present invention, gene expression levels can be compared by any method. For example, gene expression levels can be quantitatively compared based on the intensity of reporter gene expression. WT1 gene expression levels can also be quantitatively compared by RT-PCR.

In addition, the present invention can be used to compare the ratios of hepatic progenitor cells, endothelial progenitor cells, and hematopoietic progenitor cells in a number of cell populations. For example, conditions suited to concentrating desired progenitor cells can be discovered by comparing the ratios of each type of progenitor cells in cell populations fractionated under various conditions. Alternatively, given cell populations may also be induced to differentiate into desired progenitor cells under certain culture conditions. Therefore, the present invention can be used to discover culture conditions that may induce each type of progenitor cell. The development of methods for enriching the proportion of such progenitor cells is useful in acquiring more of each type of progenitor cells. Fractionation methods and culturing methods for obtaining the desired progenitor cells can be searched out using laboratory animals. Alternatively, when such research is carried out *in vitro,* methods for obtaining the desired progenitor cells can be established using human cell materials.

All prior art references cited herein are incorporated by reference.

### [Examples]

Herein below, the present invention will be specifically described using Examples, but it is not to be construed as being limited thereto.

### [Example 1]

Viable WT1-expressing cells were isolated, and then morphologically and functionally evaluated. WT470LZ transgenic (WT470LZ Tg) mice were used for this purpose (Non-Patent Document 17). These WT470LZ transgenic mice are mice into which a 470-kb yeast artificial chromosome (YAC) carrying the full length of the WT1 loci has been introduced. To trace the expression of WT1, lacZ reporter gene was introduced into exon 1 of the WT1 gene in YAC by homologous recombination in yeast. The YAC vector obtained as a result was microinjected into the pronuclei to produce WT470LZ Tg mice. The expression pattern of the lacZ gene reflected the known expression sites of the WT1 gene, as described in the literature (Non-Patent Document 17).

Viable WT1-expressing cells of the above transgenic mice were identified by FACS Gal and then collected, as described in the literature (Non-Patent Document 18). After labeling the fetal liver cells of E12.5 embryos of WT470LZ Tg and wild-type littermate mice with fluorescein di-β-D-galactoside (FDG; Molecular Probes, Eugene, Oregon, USA) (Non-Patent Document 18), cell sorting was carried out using FACS Vantage SE (Becton Dickinson Immunocytometry Systems, San Jose, CA). Dead cells were excluded from the analysis based on propidium iodide (PI) uptake (Fig. 1A, top). Viable cells (PI⁻ cells) were separated into three subpopulations according to their β-galactosidase activities (Fig. 1A). Fetal liver cells derived from WT470LZ Tg mice were classified into cells with high (lacZ++; 1.0±0.1%), intermediate (lacZ+; 11.6 ± 1.2%), and low or undetectable (lacZ-; 87.4 ± 1.2%) levels of β-galactosidase activity. The lacZ+ cells were a mixture of cells with intermediate level of WT1 expression, and cells with endogenous β-galactosidase activity. The frequency of WT1+ cells was calculated by subtracting the frequency of lacZ+ cells in the wild-type littermates from the frequency of lacZ+ cells in WT470LZ Tg mice. The frequency of WT1+ cells comprised in the lacZ+ cell population was 3.2 ± 0.5% (Table 1). Table 1 shows the frequencies of lacZ++ cells, lacZ+ cells, and lacZ- cells in the fetal liver, limb, and brain. LM refers to littermates. The frequencies of lacZ+ cells and lacZ++ cells in littermates were subtracted from the corresponding values in WT470LZ Tg mice, and the resulting values were indicated under strain item Tg-LM. The frequency of lacZ- cells shown under strain item Tg-LM was calculated by subtracting the sum of the frequencies of lacZ+ cell and LacZ++ cell in the Tg-LM strain from 100%. Each value is shown as a mean ± SE.

**Table 1**

| Cells | mouse | lacZ⁻ | lacZ⁺ | lacZ⁺⁺ |
|---|---|---|---|---|
| Fetal liver | WT470LZ Tg | 87.4 ± 1.2% | 11.6 ± 1.2% | 1.0 ± 0.1 % |
| (n=15) | Littermates | 91.6 ± 1.1% | 8.4 ± 0.9% | 0.03 ± 0% |
| | Tg-LM | 95.8± 0.5% | 3.2 ± 0.5% | 1.0 ± 0.1 % |
| Fetal brain | WT470LZ Tg | 82.2 ± 5.3% | 17.1 ± 5.1 % | 0.7 ± 0.1 % |
| (n=4) | Littermates | 92.5 ± 2.2% | 7.5 ± 2.0% | 0.03 ± 0% |
| | Tg-LM | 89.7 ± 4.3% | 9.6 ± 4.2% | 0.7 ± 0% |
| Fetal limb | WT470LZ Tg | 68.8 ± 2.5% | 31.0 ± 2.5% | 0.2 ± 0.1% |
| (n=3) | Littermates | 82.6 ± 1.8% | 17.3 ± 2.3% | 0.03 ± 0 % |
| | Tg-LM | 86.1 ± 1.2% | 13.7 ± 0.9% | 0.2 ± 0.1 % |

The present inventors examined whether or not β-galactosidase activity strictly correlates with WT1 expression level. As described in the literature (Non-Patent Document 19), lacZ++ cells, lacZ+ cells, and lacZ- cells were FACS-sorted, and the WT1 expression level in each population was measured by real time RT-PCR. The results are shown in Fig. 1B. The WT1 mRNA expression levels in lacZ++ cells, lacZ+ cells, and lacZ- cells, when the WT1 gene expression level in leukemia cell line K562 is defined as 1, were 2.21 (±1.62) x 10⁻², 3.54 (±3.39) x 10⁻⁴, and 1.94 (±1.73) x 10⁻⁴, respectively (n=4). Since approximately two thirds of the lacZ+ cells were considered to be cells that do not express WT1, the WT1 expression level of pure WT1-expressing cells present in the lacZ+ cell population was considered to be equivalent to approximately three times the expression level obtained for the lacZ+ cell population. These results showed that although lacZ+ cells contain a large number of cells having non-specific β-galactosidase activity, lacZ++ cells, lacZ+ cells, and lacZ- cells correspond to cells in which the WT1 mRNA expression level is high (WT1++), intermediate (WT1+), and low or undetectable (WT-), respectively.

FACS-sorted fetal liver lacZ++ cells, lacZ+ cells, and lacZ- cells were cytocentrifuged and May-Grunwald-Giemsa stained. The morphological characteristics of lacZ++ cells, lacZ+ cells, and lacZ- cells were remarkably different (Fig. 1C). Most lacZ++ cells had a broad basophilic cytoplasm and a nucleus with a fine chromatin structure, suggesting they were immature cells. However, they differed from hematopoietic stem cells and progenitor cells. lacZ+ cells comprised cells with non-specific β-galactosidase activity, and were partially heterogeneous. Approximately one third of lacZ+ cells were cells comprising a narrow cytoplasm and a nucleus with fine chromatin structure, and this suggested that these cells are primitive hematopoietic progenitor cells. Most of the remaining lacZ+ cells were erythroblasts at various stages of maturation. Most lacZ- cells were mature erythroblasts. These results clearly showed that mouse fetal liver cells can be distinctly separated into three different subpopulations according to WT1 gene expression level.

### [Example 2]

The present inventors examined whether or not many hepatic progenitor cells are comprised among the fetal liver cells expressing WT1. lacZ++, lacZ+, and lacZ- fetal liver cells from WT470LZ Tg mice were FACS-sorted, and hepatic colony forming units in culture (H-CFU-C) were assayed as described in the literature (Non-Patent Document 20). For every 5.0 x 10³ cells, lacZ++, lacZ+, and lacZ- fetal liver cells produced 52.7+6.1, 0.33±0.33, and zero H-CFU-C colonies, respectively (n=3) (Fig. 2A). These colonies were positively stained by anti-albumin antibodies (Intercell Technologies Inc., Hopewell, NJ), and were shown to be H-CFU-C colonies (Fig. 2B and C). These results clearly indicated that many hepatic progenitor cells are comprised among WT1+ fetal liver cells.

### [Example 3]

The present inventors investigated the frequency of endothelial progenitor cells (EPCs) present in the lacZ++, lacZ+, and lacZ- fetal liver cells. The analysis was carried out according to the literature (Non-Patent Document 21) using an EPC culture system.

FACS-sorted fetal liver cells were plated onto fibronectin-coated 35-mm culture plates at a cell density of 1.5 x 10⁴ cells per plate. The number of adherent cells produced per mm² of lacZ++, lacZ +, and lacZ- fetal liver cells derived from WT470LZ Tg mice were 43.7±14.5, 9.7±2.3, and 1.3±1.1, respectively (n=4) (Fig. 2D). Uptake of Dil-labeled acetylated low-density lipoprotein (acLDL-Dil) (Sigma-Aldrich, St. Louis, MI) was detected in the adherent cells after four hours of co-culturing, and this showed that these cells are endothelial cells (Figs. 2E and 2F). These results clearly showed that many endothelial progenitor cells are comprised among WT1++ fetal liver cells.

### [Example 4]

The present inventors also analyzed the frequencies of hematopoietic progenitor cells in lacZ++, lacZ +, and lacZ- fetal liver cells. lacZ++, lacZ +, and lacZ- fetal liver cells derived from WT470LZ Tg mice were cultured in methylcellulose medium supplemented with 15% fetal calf serum, 50 ng/mL of mouse SCF, 10 ng/mL of mouse IL-3, 10 ng/mL of human IL-6, and 3 units/mL of human Epo (Methocult 3434, Stem Cell Technologies Inc., Vancouver Canada). On average, for every 1.5 x 10⁴ cells: lacZ++ cells, lacZ+ cells, and lacZ- cells formed 0.5±0.3, 3.7±0.7, and 0.3±0.3 colony forming unit-granulocyte, erythroblast, macrophage, megakaryocyte (CFU-GEMM) colonies; 5.8±2.4, 43.7±8.2, and 9.3±4.9 colony forming unit-granulocyte/macrophage (CFU-GM) colonies; and 0.8±0.8, 4.2±2.1, and 2.8±1.4 burst-forming unit-erythroid (BFU-E) colonies, respectively (n=4, Fig. 2G). These results showed that many CFU-GEMM, CFU-GM, and BFU-E are comprised among lacZ+ fetal liver cells.

As indicated in Fig. 1A, the lacZ+ subpopulation comprised many cells with non-specific β-galactosidase activity. Therefore, the hematopoietic colony forming abilities of lacZ+ cells and lacZ- cells derived from wild type mice were analyzed to rule out the possibility that many hematopoietic progenitor cells exist among cells with endogenous β-galactosidase activity. lacZ+ cells and lacZ- cells formed zero and 3.0±1.1 CFU-GEMM colonies, 18.0±5.6 and 16.5±4.6 CFU-GM colonies, and 4.2±1.1 and 6.3±1.1 BFU-E colonies, respectively (n=3). These results showed that hematopoietic progenitor cells are not present in large numbers among cells with endogenous β-galactosidase activity, but are present in large numbers among cells with intermediate levels of WT1 expression.

### [Example 5]

The present inventors examined whether or not "WT1-expressing progenitor cells" are also present in other fetal tissues such as brain tissues and mesencymal tissues. Cell suspensions derived from fetal brain and fetal limb were prepared and analyzed by FACS-Gal assay, as described in the literature (Non-Patent Documents 22 and 23).

The frequency of lacZ++ cells in the fetal brain of WT470LZ Tg mice was 0.7±0.1% (Fig. 3A, Table 1). The frequency of WT1+ cells was calculated by subtracting the frequency of lacZ+ cells in wild-type mice from the frequency of lacZ+ cells in WT470LZ Tg mice. The frequency of WT1+ cells was 9.6±4.2% (Fig. 3A, Table 1). The frequency of lacZ++ cells in the fetal limbs of WT470LZ Tg mice was 0.21±0.07% (Fig. 3B, Table 1). The frequency of WT1+ cells was 13.7±0.9% (Table 1). These results showed that "cells expressing high levels of WT1" also exist at low frequency in fetal brain cells and mesenchymal cells. Thus, in both fetal brain and fetal limb, cells with intermediate levels of WT1 expression were more common.

WT1 expression in humans and mice shares a common pattern. For example, in hematopoietic cells, WT1 expression in undifferentiated cells is observed in both humans and mice (Fraizer, G.C., Patmasiriwat, P., Zhang, X. & Saunders, G.F. (1995) Expression of the tumor suppressor gene WT1 in both human and mouse bone marrow. Blood, 86, 47044706). Therefore, human hepatic progenitor cells, endothelial progenitor cells, and hematopoietic progenitor cells can be obtained based on the present invention.

### Industrial Applicability

The molecular markers of the present invention enable simple identification and collection of progenitor cells from tissues in which progenitor cells were not yet confirmed. In particular, since the molecular markers of the present invention are common to progenitor cells of various lineages, such as hepatic progenitor cells, endothelial progenitor cells, and hematopoietic progenitor cells, they have the advantage of being highly versatile when identifying and collecting progenitor cells.

In the future, hepatic progenitor cells may become tools for cell therapies against liver diseases such as hepatic cirrhosis. In addition, active research aimed at transdifferentiation into β cells of the pancreas, also an endodermal organ, is also underway. Treatment using vascular endothelial progenitor cells is already being performed at the clinical trial level, and WT1-positive endothelial cells, which are a different cell population from the vascular endothelial progenitor cells currently in use, may be even more useful.

## Claims

1. A method for separating a hepatic, endothelial, or hematopoietic progenitor cell from a cell population, wherein the method comprises the steps of:
a) detecting the expression of a WT1 gene in a cell in a cell population; and
b) separating the cell in which expression of the WT1 gene was detected.

2. A method for simultaneously separating at least two progenitor cells from a cell population, wherein the progenitor cells are selected from hepatic, endothelial, and hematopoietic progenitor cells, and wherein the method comprises the steps of:
a) detecting the expression of a WT1 gene in a cell in a cell population comprising at least two progenitor cells, selected from hepatic, endothelial, and hematopoietic progenitor cells; and
b) separating the cells in which expression of the WT1 gene was detected.

3. The method of claim 1 or 2, wherein expression of the WT1 gene is detected by using expression of a WT1 gene or of a reporter gene linked to a WT1 promoter as an indicator.

4. The method of claim 3, wherein the reporter gene is a lacZ gene or GFP gene, and expression of the reporter gene is detected by a FACS assay.

5. The method of any one of claims 1 to 4, wherein a hepatic progenitor cell or an endothelial progenitor cell is separated when the expression level of the WT1 gene is in the range of 2.21 (±1.62) x 10⁻² (when expression of the WT1 gene in a K562 leukemia cell line is defined as 1), and a hematopoietic progenitor cell is separated when the expression level of the WT1 gene is in the range of 3.54 (±3.39) x 10⁻⁴ (when expression of the WT1 gene in a K562 leukemia cell line is defined as 1).
